# EUROPEAN PATENT APPLICATION

(11) **EP 4 318 414 A1**
(43) Date of publication of application: **07.02.2024**
(21) Application number: 22188258.2
(22) Date of filing: 02.08.2022
(51) Int. Cl.: G06V 40/10, G06V 10/82, A61B 5/00, G06N 3/045, G16H 30/40

(54) **DETECTION OF SAFETY STATES DURING MEDICAL IMAGING PROCEDURES**

(71) Applicant: Koninklijke Philips N.V., 5656 AG Eindhoven (NL)
(72) Inventor: KRUEGER, Sascha, Eindhoven (NL); KOKEN, Peter, Eindhoven (NL); SOMMER, Karsten, 5656AG Eindhoven (NL); KNOESTER, Jaap, 5656AG Eindhoven (NL)
(74) Representative: Philips Intellectual Property & Standards

(57) **Abstract**

Disclosed herein is a medical system (100, 300). The execution of machine executable instructions (120) causes a computational system to (104): receive (200) an overview image (128), receive (202) object locators (130) in response to inputting the overview image into a first neural network (122), construct (204) an object sub image (132) using the object locator, receive (206) object positional data (134) in response to inputting the object sub image for each of the multiple predetermined objects into an object specific neural network (124), construct (208) multiple search images (136) using the object positional data for the multiple predetermined objects, receive (210) locator data (138) descriptive of a position of an item in response to inputting each of the multiple search images into an item specific neural network (126), detect (212) a safety state (140) by comparing the locator data and the object positional data of each of the predetermined objects with respect to a predetermined criterion; and provide (214) a warning signal (144) if the safety state is detected

## Description

### FIELD OF THE INVENTION

The invention relates to medical imaging, in particular to safety during medical imaging procedures.

### BACKGROUND OF THE INVENTION

Various medical imaging techniques such as Magnetic Resonance Imaging (MRI), Computed Tomography, Positron Emission Tomography, and Single Photon Emission Tomography enable detailed visualization of anatomical structure of a subject. In medical imaging modalities such as these, the proper placement of the subject prior to the procedure is critical. Otherwise the subject could be improperly imaged.

United States patent application publication US 20213/012659 A1 discloses a medical instrument that comprises a medical imaging system configured for acquiring medical imaging data from a subject, a subject support configured for supporting the subject during acquisition of the medical imaging data, and an optical imaging system configured for acquiring optical imaging data of the subject on the subject support. The execution of the machine executable instructions causes a processor controlling the medical instrument to: control the optical imaging system to acquire the optical imaging data, generate the initial vector using the optical imaging data, generate the synthetic image by inputting the initial vector into a generator neural network, calculate a difference between the synthetic image and the optical imaging data, and provide a warning signal if the difference differs by a predetermined threshold. The generator neural network is trained to generate a synthetic image of the subject on the subject support in response to inputting an initial vector.

### SUMMARY OF THE INVENTION

The invention provides for a medical system, a computer program, and a computer-implemented method in the independent claims.

The use of a camera system to monitor the setup and safety of a medical imaging system may have several challenges. One challenge is that if the system has even a slight error rate the operators are likely to disable it. To be useful in a clinical situation such a system needs to have near perfect accuracy. Another difficulty is that it can be very labor intensive to properly train the neural networks used to monitor the images.

Embodiments may have the advantage of having a high accuracy as well as a being able to be modified to a particular clinical situation of medical imaging protocol easily. Embodiments may possibly achieve this by using a hierarchy of neural networks as exemplary described in the following: first, an overview image is received. A first neural network first provides object locators to identify a predetermined list of objects in a lower resolution version of the overview image. These object locators are then used to construct object sub images from the overview image which are then fed into an object specific neural network. The object sub images have an intermediate resolution. The object locators are then, and possibly with additional information, to construct multiple search images from the overview image. Specialized item specific neural networks are then used to search for specific items or safety conditions in the multiple search images. The use of the hierarchy of neural networks may provide for neural networks that can be accurately trained for specific tasks. The hierarchical structure may also enable item specific neural networks and object specific neural networks to be added or removed from the system easily.

In one aspect the invention provides for a medical system that comprises a memory storing machine-executable instructions. The memory further stores a first neural network, an object-specific neural network, and an item-specific neural network. The medical system further comprises a computational system. Execution of the machine-executable instructions causes the computational system to receive an overview image. The overview image has an original resolution and is descriptive of a subject position on a subject support of a medical imaging system. Execution of the machine-executable instructions further causes the computational system to receive object locators descriptive of a positive and orientation of multiple predetermined objects in the overview image in response to inputting the overview image into a first neural network.

The first neural network is configured to process the overview image at a first resolution. The first resolution is lower than the original resolution. The multiple predetermined objects comprise the subject and the subject support. Execution of the machine-executable instructions further causes the computational system to construct an object sub-image for each of the multiple predetermined objects using the object locator. Each object sub-image has a second resolution. The second resolution is lower than the original resolution and higher than the first resolution. Execution of the machine-executable instructions further causes the computational system to receive object positional data descriptive of each of the multiple predetermined objects in response to inputting the object sub-image for each of the multiple predetermined objects into an object-specific neural network.

The object-specific neural network is unique for each of the multiple predetermined objects. Execution of the machine-executable instructions further causes the computational system to construct multiple search images using the object positional data for the multiple predetermined objects. The multiple search images have a third resolution that is higher than the second resolution. The third resolution is less than or equal to the original resolution. Execution of the machine-executable instructions further causes the computational system to reserve locator data descriptive of a position of an item in response to inputting each of the multiple search images into an item-specific neural network. Execution of the machine-executable instructions further causes the computational system to detect a safety state by comparing the locator data and the object positional data of each of the predetermined objects with respect to a predetermined criterion. Execution of the machine-executable instructions further causes the computational system to provide a warning signal if the safety state is detected.

The safety state may for example be a flag in software or label that is set when the predetermined criterion is met. In one concrete example the multiple search images could define regions or areas where it is not advantageous for a subject to place his or her hands. The multiple search images could be searched using an item-specific neural network that is specifically trained to locate hands. The use of an item-specific neural network if it detects a hand would then set a safety state indicating that a particular search image has a hand located in it. The warning signal could take different forms in different examples. For example, it may cause a graphical user interface to display a warning or additional information. In other cases, the warning signal could be used to change the behavior of the medical system. For example, it may cause the medical system to pause functioning until given a command by an operator. In other cases, it may disable various functions such as moving a subject support. The warning signal may be integrated into a control loop or a control system.

Embodiments may be beneficial because there is a hierarchy of neural networks that is provided. For example, using the previous example, the identification of hands being in a place which may be dangerous for a subject may be difficult to train with a single neural network. The first neural network operates at a lower resolution and is able to identify objects in general or label them. This is a task which is well known and performed very well by various neural networks. It is also possible to train neural networks by general labeling within an image. The next level is an object-specific neural network.

This may actually be one neural network or a combination of neural networks that work together to look at each object specifically using a neural network trained for that specific object. This is then used to provide detailed information about the orientation or the object positional data. This object positional data can be used to then define the position of multiple search images. Sometimes the multiple search images may be defined in terms of the object positional data from one object or it may be from multiple objects. For example, in one example, object positional data from two objects could be used to trace wires going between the two objects or ensuring that they have a certain amount of distance between each other. In the case of a subject support, it could be that the locations on a specific subject support could be used to define the multiple search images.

The various image resolutions for the hierarchy of neural networks could be provided in a variety of ways. For example, there may be intermediate steps where the resolution of the various images is reduced performing an averaging process. In other examples, the neural networks could do this themselves with an averaging max pooling layer at the input. That is to say, for example, like the first neural network may have an averaging max pooling layer that reduces the overview image from the original resolution to the first resolution.

The overview image may take different forms in different examples. In one example the overview image is a conventional image from a camera or video system. The conventional image could for example be an RGB or greyscale image. In this case, the going between the first resolution, the second resolution, and the third resolution involves a transformation or reduction of resolution of the overview image when constructing images that are input into the neural networks. As was mentioned above this reduction of the resolution can be done at the time an image is reconstructed or it can be done by pooling layers at the input to one of the neural networks.

Another possibility is that the overview image comprises composite data from multiple image modalities or sensor types. For example, the camera or video system may provide a three-dimensional image along with an RBG or greyscale image. In this case the image would have more than three channels of data. It would have the three RGB channels plus an additional channel with depth data. In this case the neural networks would be configured to accept 4 channels of data instead for three for input. The reduction of resolution to the first resolution, the second resolution, and the third resolution is understood to apply to all channels of the overview image.

As is mentioned above, the object-specific neural network could be a single neural network or a chain of neural networks. In another embodiment the multiple search images may be defined by dedicated specific system risk areas. These dedicated specific system risk areas may be defined in terms of relative coordinates to the object positional data for specific objects.

In some specific examples the warning signal may be used to halt the automated setup of a medical imaging system. For example, it may cause subject support motion to stop when fingers are in an unsafe location or pose. Another example would be to operate the table or subject support when a collision is likely. For example, if there is a likely collision the table or subject support could be operated such that it has a velocity where it is less likely to injure someone.

In a further example, the warning signal may cause the medical system to modify its behavior such that it only allows manual override by controls that are close to the patient. For example, there may be a control which is used to insert the subject into a medical imaging system. The warning signal may force or modify the system such that only controls which are adjacent to the patient or subject are allowed to move the subject. This would reduce the changes that an operator who is more distant would cause injury to the subject.

An advantage of using a hierarchy of neural networks is that specialized neural networks are more specific and sensitive for a given capacity. This may have the benefit of increasing the accuracy of the overall system and reducing the occurrence of false alarms. Also, neural networks that are trained individually against individual targets are often complementary Another advantage is that additional objects and things can be added to the system without modifying the detection performance of existing object detection networks. The first neural network would be trained to recognize all the objects that are currently in use but then under this, the specific neural networks in the hierarchy, once they are trained, would not need to be retrained.

The neural networks used in this medical system, this includes the first neural network, the object-specific neural network and the item-specific neural network can be implemented in different architectures. Example architectures of a neural network that may be used may include elements of a ResNet, a U-net, and fully convolutional layers in general. Such fully convolutional neural networks may be particularly beneficial because they are especially suitable for detection and localization of individual objects against the background of a multitude of other objects, i.e. have shown to have very good sensitivity and specificity.

The first neural network can be trained with images with the first resolution that have been labeled with the multiple predetermined objects (labels may include outlines, segmentation masks, keypoints or bounding boxes). The object specific neural network may be trained with images with the second resolution that have been labeled with positional data for the specific object the object specific neural network is assigned to. The item specific neural network may be trained with images with the third resolution that have been trained with the specific items.

In another embodiment the use of the predetermined criterion may be used to provide a hierarchy of security hazards and risks. For example, the predetermined criterion may provide a ranking for severity of an event. For example, there could be multiple classes of security hazards such as a normal security hazard and a high severity security hazard. The basic response of the medical system could have its functioning modified according to these two classes.

In some examples the warning signal may be displayed or presented to the user a visual user interface or other audible system. The warning signal may for example be used to control or restrict the use of the machine, for example if there is a dangerous situation the medical system may be disabled or disabled temporarily. Each finding or security risk that the system identifies may for example be assigned a different type or a different level of warning. As is mentioned above, the warning signal may also be a control signal for the medical system.

In another embodiment execution of the machine-executable instructions further causes the computational system to display a warning graphical user interface if the warning signal is provided. The warning graphical user interface is configured to perform any one of the following: provide a spatial localization of the cause of the safety state, display a warning signal type and/or warning context for using the predetermined criterion, display a severity ranking using the predetermined criterion, display operator instructions reviewed from an instruction database in response to a query using the predetermined criterion, provide a voice or audible alert, project a localized warning light onto the subject using a projector and combinations thereof. When the localized warning light is configured to, maybe in some examples, be configured to illuminate the item that triggered the safety state, for example, if the subject's hands are at risk of being crushed or pinched, then the light may shine onto the hands and this may provide an improved means of ensuring that either the subject and/or the operator place the subject's hands in a safer location.

In another embodiment the object-specific neural network for the subject is configured to output a set of anatomical keypoint locators. At least a portion of the multiple search images are determined using a predetermined geometric relation or position relative to to the set of anatomical keypoint locators. For example, in the case of testing when the subject places his or her hands in a location where the subject may be likely to cause a conductive loop the various anatomical locations where the subject could put the hand in a disadvantageous position can be defined relative to the anatomical set of keypoint locators. For example, the location of the multiple search images may be defined in terms of coordinates of the set of anatomical keypoint locators. Once the set of anatomical keypoint locators are determined from the object-specific neural network it may then use these to calculate the location of the multiple search images.

An anatomical keypoint as used herein encompasses an anatomical landmark or position located in the subject. Often times the anatomical keypoint locator is referred to as joint locations. However, the anatomical keypoint is broader than the location of a joint. Anatomical keypoints could incorporate the locations of various joints or anatomical parts of the subject. The camera image is descriptive of a subject and provides a description of the exterior or surface of the subject. For example, the camera image could be an optical image, an infra-red image, or even a color image. In other examples the camera image is a three-dimensional surface image.

In another embodiment execution of the machine-executable instructions further causes the computational system to filter the locator data using a locator filter modified by the keypoint locators. Execution of the machine-executable instructions further causes the computational system to ignore locator filter data filtered by the locator filter. In this embodiment the system can be programmed to ignore locator data that is defined by particular keypoint locators. This for example may be very useful in ignoring data from an operator as opposed to a subject. For example a subject may be reposed on subject support or table and an operator may be working on configuring the medical system. The data relative to the operator may then be ignored or handled separately.

In another embodiment the item is a nurse call squeeze bulb. The use of a specialized neural network to identify this may be beneficial because often times the nurse call squeeze bulb is located within a subject's hand and may otherwise be difficult to locate using a general neural network.

In another embodiment the item is a headset and/or earplugs. The multiple search images comprise a head region of the subject. The locator data is descriptive of a position of the item relative to a center locator of the ears of the subject.

In another embodiment the item is a conductive cable. The multiple predetermined objects comprise a magnetic resonance imaging coil and/or a wired apparatus. A wired apparatus herein is a device which is connected to a control or communication wire. The multiple predetermined objects further comprises at least one magnetic resonance transmit body coil element. The multiple search images comprise regions adjacent to the magnetic resonance imaging coil and/or the wired apparatus. The multiple search images further comprise regions adjacent to the at least one transmit body coil element. The multiple search regions further comprise additional regions with a predetermined location relative to the set of anatomical keypoint locators. This embodiment may be beneficial because it may enable keeping the cables to a magnetic resonance imaging coil or wired apparatus away from locations which may wind up causing a radio frequency burn on the subject.

In another embodiment the item is a hand. The warning signal is configured to provide a dangerous hand position warning signal if the hand is located at a pinch location or a current loop-causing position. A current loop-causing position as used herein encompasses a position where the subject has placed his or her hand such that a conductive loop is formed by the subject's body and arm. During a magnetic resonance imaging examination, this may cause a current or locally increased SAR exposure induced by the radio frequency field to cause heating and/or a burn of the subject.

The multiple search images may be chosen to look at specific danger or high-risk positions. They may identify high resolution areas using a model of the body and/or identification of other objects. The search-defined areas or the multiple search images may be defined in terms of whether it would be dangerous to put a hand on a subject support or in relation to anatomy such as a hip area.

In another embodiment the item is a magnetic resonance conditional device such as a device which is allowed for a specific set of magnetic resonance imaging protocol parameters or for specific locations with respect to the MRI system. The locations where the magnetic resonance conditional device is allowed may be derived from specified cleared use cases for particular magnetic resonance imaging protocols. This may include such things as keeping the device within a B0 field of a certain strength, and do search images to search images that cover up to a certain magnetic isoline. In other instances, this may involve ensuring that certain distance between different devices is kept and the search images may cover the problem area in between or between these devices.

In another embodiment the multiple predetermined objects comprise one or more magnetic resonance imaging coils. The locator data comprises radio frequency cable location data. Execution of the machine-executable instructions further causes the computational system to determine the safety state performing any one of the following: comparing the object positional data of the one or more magnetic resonance imaging coils and the radio frequency location data to a set of allowable radio frequency routing paths, comparing the object positional data of the one or more magnetic resonance imaging coils with the set of reported magnetic resonance imaging coil connections and combinations thereof. The reported magnetic resonance imaging coil connections may for example be supplied by the radio frequency system of the magnetic resonance imaging system which is able to detect if a coil is attached or not.

In another embodiment the overview image is an optical image.

In another embodiment the overview image is a color optical image.

In another embodiment the overview image is a three-dimensional image. For example, a so-called three-dimensional camera may be used to acquire the three-dimensional image.

In another embodiment the medical system further comprises a medical imaging system with a subject support. The medical system further comprises an imaging device configured to acquire the overall image by imaging at least the subject support. Execution of the machine-executable instructions further causes the computational system to control the imaging device to acquire the overview image.

In another embodiment the medical imaging system is a magnetic resonance imaging system.

In another embodiment the medical imaging system is a computed tomography system.

In another embodiment the medical imaging system is a combined magnetic resonance imaging and computed tomography system.

In another embodiment the medical imaging system is a positron emission tomography system.

In another embodiment the medical imaging system is a combined magnetic resonance imaging system and computed tomography system.

In another embodiment the medical imaging system is a single photon emission tomography system.

In another embodiment the medical imaging system is a digital X-ray system.

In another embodiment the medical imaging system is a digital fluoroscopy system.

In another embodiment the medical imaging system is a magnetic resonance imaging-guided radiotherapy system.

In another embodiment the medical imaging system is a computed tomography-guided radiotherapy system.

In another embodiment the medical imaging system is a magnetic resonance imaging-guided high-intensity focused ultrasound system.

In another embodiment the medical imaging device is a three-dimensional camera.

In another embodiment execution of the machine-executable instructions further causes the computational system to compare spatial contours of the item to a predetermined volume to detect a collision condition.

In another embodiment execution of the machine-executable instructions further causes the computational system to use contour data from the three-dimensional camera to filter out and ignore locator data as being non-examination setup related. For example, the three-dimensional camera can be used to determine or identify objects which are outside of a zone of the subject support, i.e. not affected by the subject transport mechanics (equipment, operator).

In another embodiment execution of the machine executable instructions further causes the computational system to determine a signal motion correlation with subject support motion to filter out and ignore locator data as being non-examination setup related. For example, when the subject support is being moved, the portions of the image which are moving can be detected in the three-dimensional image. The areas outside the moving area may then be identified as being non-examination setup related.

In another embodiment the multiple predetermined objects comprise an operator. An operator as used herein encompasses a human who is operating or controlling the medical system. Execution of the machine-executable instructions further causes the computational system to identify an operator zone within the overview image using the object locator of the operator. Execution of the machine-executable instructions further causes the computational system to separate locator data and/or object positional data within the operator zone. This may for example be useful in cases where anatomical structures of the subject are searched for but it is desirable to ignore those of the operator. For example, one would like to ignore the position of the operator's hand when determining if the subject is at risk of having his or her fingers pinched.

In another embodiment the overview image is received as an image stream with a frame rate. The first neural network is configured to process the overview image equal to or at a higher rate than the frame rate. Execution of the machine-executable instructions further causes the computational system to repeatedly input the overview image to obtain the object locators at the frame rate. In this embodiment the first neural network is operating at a low enough resolution that the images are being able to be processed at a rate equal to or greater than the frame rate. This means that essentially the first neural network is able to identify objects in a real time basis.

In another aspect the invention provides for a computer program comprising machine-executable instructions, a first neural network, an object-specific neural network, and an item-specific neural network. Execution of the machine-executable instructions causes the computational system to receive an overview image. The overview image has an original resolution and is descriptive of a subject position on a subject support of a medical imaging system. Execution of the machine-executable instructions further causes the computational system to receive object locators descriptive of a position and orientation of multiple predetermined objects in the overview image in response to inputting the overview image into the first neural network. The first neural network is configured to process the overview image at a first resolution. The first resolution is lower than the original resolution. The multiple predetermined objects comprise the subject and the subject support.

Execution of the machine-executable instructions further causes the computational system to construct an object sub-image for each of the multiple predetermined objects using the object locator. Each object sub-image has a second resolution. The second resolution is lower than the original resolution and higher than the first resolution. Execution of the machine-executable instructions further causes the computational system to receive object positional data descriptive of each of the multiple predetermined objects in response to inputting the object sub-image for each of the multiple predetermined objects into an object-specific neural network that is unique for each of the multiple predetermined objects.

Execution of the machine-executable instructions further causes the computational system to construct multiple search images using the object positional data for the multiple predetermined objects. The multiple search images have a third resolution that was higher than the second resolution. The third resolution is less than or equal to the original resolution. Execution of the machine-executable instructions further causes the computational system to receive locator data descriptive of a position of an item in response to inputting each of the multiple search images into an item-specific neural network. Execution of the machine-executable instructions further causes the computational system to determine a safety state by comparing the locator data and the object positional data of each of the predetermined objects with respect to a predetermined criterion. Execution of the machine-executable instructions further causes the computational system to provide a warning signal if a safety state is detected.

In another aspect the invention provides for a computer-implemented method. The method comprises receiving an overview image. The overview image has an original resolution and is descriptive of a subject position on a subject support of a medical imaging system. The method further comprises receiving object locators descriptive of a position and orientation of multiple predetermined objects in the overview image in response to inputting the overview image into a first neural network. The first neural network is configured to process the overview image at a first resolution. The first resolution is lower than the original resolution. The multiple predetermined objects comprise the subject and the subject support.

The method further comprises constructing an object sub-image for each of the multiple predetermined objects using an object locator. Each object sub-image has a second resolution. The second resolution is lower than the original resolution and higher than the first resolution. The method further comprises receiving object positional data descriptive of each of the multiple predetermined objects in response to inputting the object sub-image for each of the multiple predetermined objects into an object-specific neural network that is unique for each of the multiple predetermined objects. The method further comprises constructing multiple search images using the object positional data for the multiple predetermined objects.

The multiple search images have a third resolution that is higher than the second resolution. The third resolution is less than or equal to the original resolution. The method further comprises receiving locator data descriptive of a position of an item in response to inputting each of the multiple search images into an item-specific neural network. The method further comprises detecting a safety state by comparing the locator data and the object positional data for each of the predetermined objects with respect to a predetermined criterion. The method further comprises providing a warning signal if a safety state is detected.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Medical imaging data is defined herein as being recorded measurements made by a medical imaging system that is descriptive of a subject. The medical imaging data may be reconstructed into a medical image. A medical image is defined herein as being the reconstructed two- or three-dimensional visualization of anatomic data contained within the medical imaging data. This visualization can be performed using a computer.

K-space data is defined herein as being the recorded measurements of radio frequency signals emitted by atomic spins using the antenna of a Magnetic resonance apparatus during a magnetic resonance imaging scan. Magnetic resonance data is an example of medical imaging data.

A Magnetic Resonance Imaging (MRI) image or MR image is defined herein as being the reconstructed two- or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 shows a flow chart which illustrates a method of using the medical system of Fig. 1;
Fig. 3 illustrates a further example of a medical system;
Fig. 4 shows a flow chart which illustrates a method of using the medical system of Fig. 4;
Fig. 5 illustrates an example of an object sub-image that shows a subject reposing on a table trolley for a magnetic resonance imaging system
Fig. 6 shows an example of a search image;
Fig. 7 depicts an image similar to that illustrated in Fig. 5. In this example there is a status signal that is a warning that indicates a finger pinching risk detected for the left hand;
Fig. 8 illustrates the correct detection of patient pose not fitting the available examination space; and
Fig. 9 illustrates the detection of spurious volumes that are not part of the patient examination setup.

### DETAILD DESCRIPTION OF EMBODIMENTS

Like numbered elements in these figures are either equivalent elements or perform the same function. Elements which have been discussed previously will not necessarily be discussed in later figures if the function is equivalent.

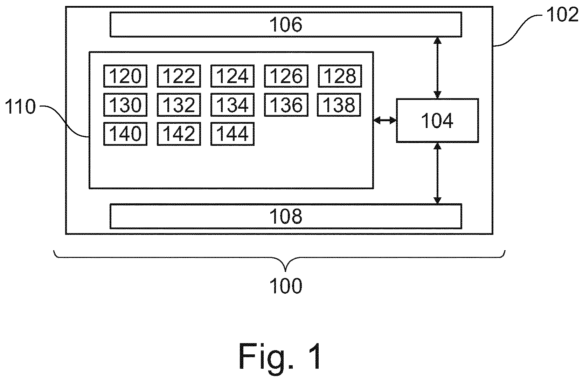

Fig. 1 illustrates an example of a medical system 100. The medical system 100 is shown as comprising a computer 102. The computer 102 may represent one or more computers at one or more locations. The medical system 100 is further shown as comprising a computational system 104. The computational system 104 may likewise represent one or more computational systems or computing cores located in one or more computers at one or more locations. The computational system 104 is shown as being in communication with an optional hardware interface 106 and an optional user interface 108. The hardware interface 106 may enable the computational system 104 to control and communicate with other components such as a medical imaging system. The user interface 108 may enable an operator or controller to control and operate the functions of the medical system 100. The computational system 104 is further shown as being in communication with a memory 110. The memory 110 may represent different types of memory which may be accessible to the computational system 104. In some examples the memory 110 is a non-transitory storage medium.

The memory 110 is shown as containing machine-executable instructions 120. The machine-executable instructions 120 enable the computational system 104 to perform basic data analysis, mathematical, image processing and control commands. The memory 110 is further shown as containing a first neural network 122, an object-specific neural network 124, and an item-specific neural network 126. The memory 110 is further shown as containing an overview image 128. The overview image 128 depicts a subject position on a subject support of a medical imaging system. The overview image 128 has an original resolution.

The first neural network 122 is configured to receive the overview image 128 and process it at a first resolution. For example, the overview image 128 may have its resolution reduced to the first resolution before it is input into the first neural network 122 or pooling layers may be used to automatically reduce the resolution before it is processed by the first neural network 122. In response to receiving the overview image 128 as input, the first neural network 122 outputs a number of object locators 130. The object locators for example may be an identifier position of multiple predetermined objects in the overview image. This may for example be the placing of bounding boxes or other segmentations to identify the location of various objects.

The memory is further shown as containing object sub-images 132 that were created from the overview image 128 using the object locators 130. For example, if object locators 130 are bounding boxes the object sub-images 132 may be constructed so that they are identical with or encompass or include the bounding boxes. The object sub-images 132 have a second resolution that is lower than the original resolution and higher than the first resolution. The memory 110 is further shown as containing object positional data 134 that was received from the object-specific neural network 124 in response to receiving the object sub-images 132. The object-specific neural network 124 is programmed to identify specific objects.

The memory 110 is further shown as containing multiple search images 136 that have been constructed using the object positional data 134. The object positional data 134 may for example contain coordinates of various portions or parts of the particular object. These can be used to identify and define the multiple search images 136. The memory 110 is further shown as containing locator data 138 that has been received in response to inputting the multiple search images 136 into the item-specific neural network 126. The item-specific neural network 126 is configured for identifying or locating a very specific item such as a hand bulb, hand, wire, or other piece of apparatus. The memory is further shown as containing a safety state 140 which is identification of a specific item that was identified in one of the multiple search images 136 using the locator data 138. The safety indicator 140 can be compared to a predetermined criterion 142. If the predetermined criterion 142 is met then a warning signal 144 may be provided. This may be provided to the operator or it may be provided to the computational system to modify the behavior of the medical system.

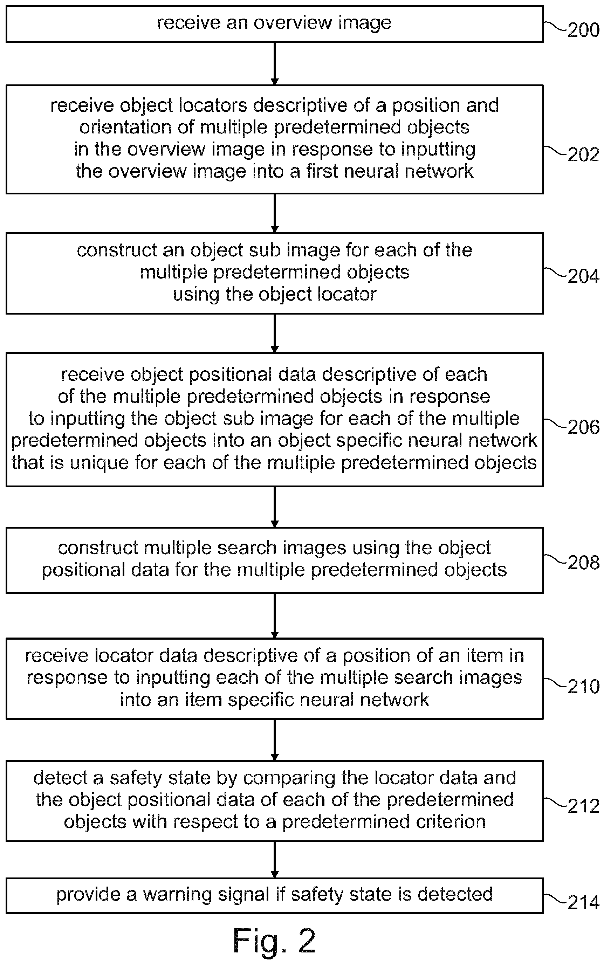

Fig. 2 shows a flowchart which illustrates a method of operating the medical system 100 of Fig. 1. First, in step 200, the overview image is received. This may include acquiring it with a camera or imaging device. Next, in step 202, the object locators are received in response to inputting the overview image 128 into the first neural network 122. Then, in step 204, the object sub-images 132 are constructed for each of the multiple predetermined objects using the object locators 130. Next, in step 206, object positional data is received from the object-specific neural network in response to inputting the object sub-image 132 for each of the multiple predetermined objects. Next, in step 208, the multiple search images 136 are constructed using the object positional data 134. Next, in step 210, locator data 138 is received from the item-specific neural network in response to inputting the multiple search images 136. Then, in step 212, the safety state 140 is detected by comparing the locator data and the object positional data of each of the predetermined objects with respect to the predetermined criterion 142. Finally, in step 214, the warning signal 144 is provided if the safety state is detected.

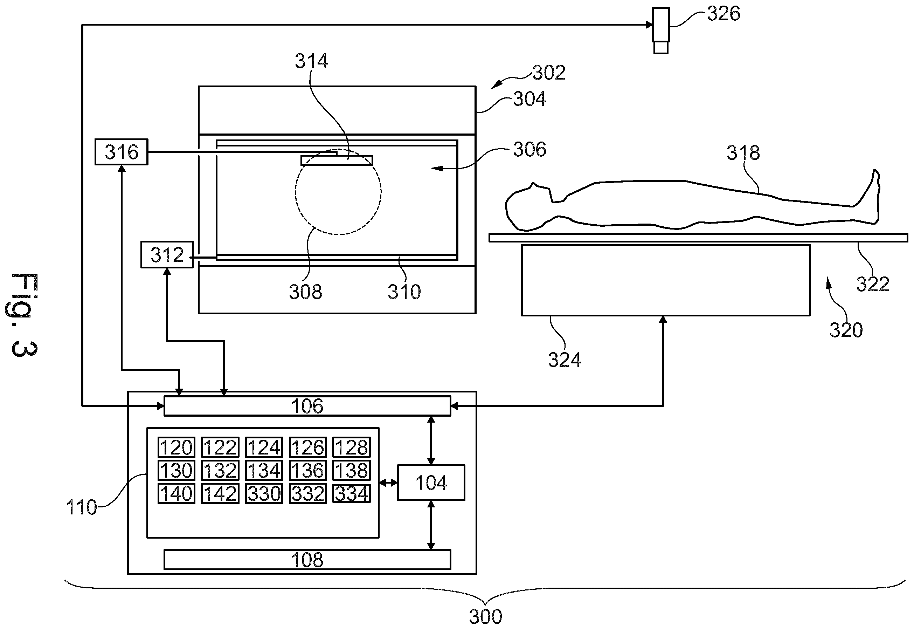

Fig. 3 illustrates a further example of the medical system 300. The example in Fig. 3 is similar to that as illustrated in Fig. 1 except that it additionally comprises a magnetic resonance imaging system 302 and an imaging device 326. The magnetic resonance imaging system 302 that is controlled by the computational system 104.

The magnetic resonance imaging system 302 comprises a magnet 304. The magnet 304 is a superconducting cylindrical type magnet with a bore 306 through it. The use of different types of magnets is also possible; for instance, it is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view may be within the imaging zone 308. The k-space data that is acquired typically acquired for the field of view. The region of interest could be identical with the field of view or it could be a sub volume of the field of view. A subject 318 is shown as being supported by a subject support 320 such that it may move at least a portion of the subject 318 within the imaging zone 308.

Within the bore 306 of the magnet there is also a set of magnetic field gradient coils 310 which is used for acquisition of preliminary k-space data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically, magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise, the transceiver 316 may also represent a separate transmitter and receivers. The radio-frequency coil 314 may also have multiple receive/transmit elements and the radio frequency transceiver 316 may have multiple receive/transmit channels.

The imaging device 326 is positioned over the subject 318 and the subject support 320 and is able to acquire the overview image 128. The subject support 320 is shown as comprising a table trolley 322 and a main motorized subject support 324. The main motorized subject support 324 is configured to receive the table trolley 322 and then insert it, with the subject 318, into the bore of the magnet 304. The transceiver 316, the gradient controller 312, the main motorized subject support 324, and the imaging device 326 are shown as being connected to the hardware interface 106 of the computer system 102.

The memory 110 is further shown as containing pulse sequence commands. The pulse sequence commands are commands or data which may be converted into commands which enable the magnetic resonance imaging system 302 to acquire magnetic resonance data from the imaging zone 308. For example, if the subject 318 were inserted into the magnet 304 k-space data could be acquired from the imaging zone 308. The memory 110 is further shown as containing measured k-space data 332 that had been acquired by controlling the magnetic resonance imaging system 302 with the pulse sequence commands 330. The memory 110 is further shown as containing magnetic resonance data 334 that has been reconstructed from the measured k-space data 332.

In Fig. 3 the magnetic resonance imaging system 302 is used as an example of a medical imaging system. However, different medical imaging systems could be substituted in place of the magnetic resonance imaging system illustrated in Fig. 3. For example, the magnetic resonance imaging system could be replaced with any one of the following: a computed tomography system, a combined magnetic resonance imaging and computed tomography system, a positron emission tomography system, a combined magnetic resonance imaging and computed tomography system, a single photon emission tomography system, a digital X-ray system, a digital fluoroscopy system, a magnetic resonance imaging guided radiotherapy system, a computed tomography guided radiotherapy system, and a magnetic resonance imaging guided high intensity focused ultrasound system.

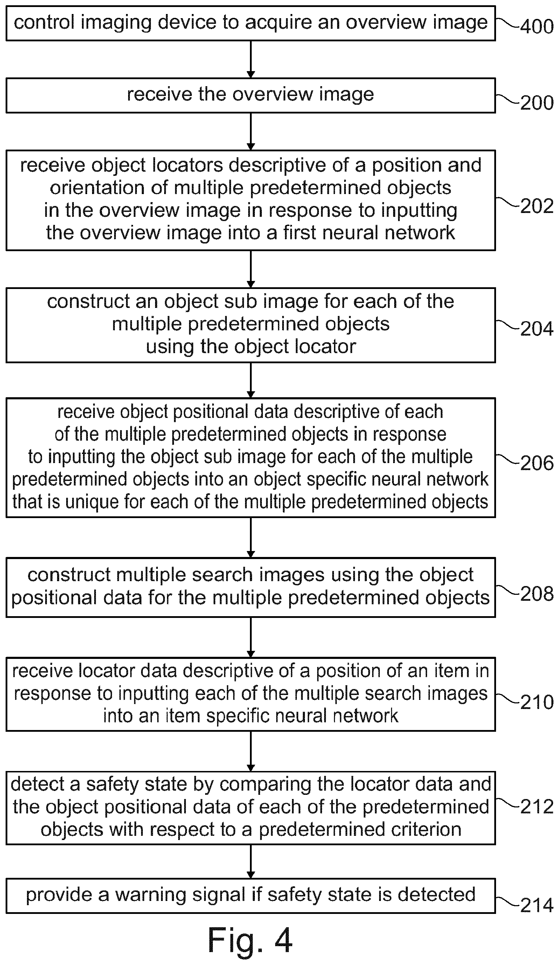

Fig. 4 shows a flow chart which illustrates a method of using the medical system 300 of Fig. 3. In step 400 the imaging device 326 is controlled to acquire the overview image 128. After this, the method proceeds to steps 200, 202, 204, 206, 208, 210, 212, and 214. Additional steps may also be performed after this. For example, the main motorized subject support 324 may be used to insert the subject into the bore 306 of the magnetic resonance imaging system 302. After this the pulse sequence commands may be used to control the magnetic resonance imaging system to acquire the measured k-space data 332. Finally, the measured k-space data may be reconstructed into the magnetic resonance image 334.

Preparing a radiology (e.g. MR, CT, Xray) system (medical system 100, 300) and patient (subject 318) for an examination is a time-consuming task and requires trained skills of the operator. An AI-based body and object keypoint detection using a ceiling-mounted table-facing camera (imaging device 326) may be used to provide guidance to the operator and automate this task. Safe-guarding the examination preparation is of strong interest in this context since it represents a main enabler for automatization and autonomous or even remote scanner operation. Especially moving the patient in and out of the bore is currently associated with safety risks such as collision and finger pinching.

Today, both patient (subject 318) preparation and movement of the patient into the bore are performed with the operator standing at either side of the patient support. Examination setups are typically complex and consist of multiple layers of immobilization, comfort and scanning devices. Therefore, it is not surprising that given the limited view of the operator and the scheduling time pressure, many safety incidents are reported.

Examples may comprise one or more of the following features:
- An RGB/Depth camera (imaging device 326) overviewing the exam preparation area that provides a continuous data stream (overview image 128) of the patient examination preparation area incl. patient table and adjacent area
- Dedicated set of neural networks (first neural network 122, object specific neural network 124, and item specific neural network 126) for detection of body parts and objects in the camera images
- Dedicated set of specific system risk areas (as defined in the multiple search images 136)
- Algorithms to filter operator-caused signals from relevant patient-caused signals
- An algorithm for detection of unsafe actions or configurations
- An algorithm to classify the severity of the event into at least two classes (e.g. normal and high severity)
- Feedback via user interface to the operator and automatic system reaction (warning signal 144) to prevent safety incidents

Examples may use camera sensors (imaging device 326) to monitor the examination room in combination with algorithms to detect specific people, objects and actions that are associated with safety risks and to provide actions to prevent them. Specifically usage of a cascade of neural networks for local investigation of small and subtle objects (nurse call, cable parts, connector etc.), body parts (e.g. finger joints) and specific system risk areas (table-dock and -bore junctions, trolley dock etc.) is proposed.

In one example, an overview network (first neural network 122) detects the main patient keypoints (i.e. anatomical landmarks such as joints) which then allows to run subsequent networks (item specific neural network 126) locally (in the multiple search images 136) at maximum resolution (between the original resolution and the third resolution) for closer investigation of respective body subregions.

In the above the first neural network 122 provides the keypoints. An alternative to this is that the first neural network 122 provides object locators 130 (for example bounding boxes) for the predetermined multiple objects. These bounding boxes are then used to make the object sub images which then do detailed processing on each object. For example, the objects specific neural network outputs keypoint data as the object positional data.

In an exemplary case, a specific safety risk is associated with fingers grabbing around the table edges during table movements into or out of the bore. Specifically, locations like the junction to the table support motor stage are potentially dangerous locations. To further improve safety usage of a two-pronged approach is proposed, where next to the detection of body and fingers, additionally, the risk areas are frequently monitored at highest possible resolution. This significantly may improve the detection capability since it strengthens the detection in case of obstructions or when the patient is moving out of the bore after scanning. In another exemplary case, the examination setup may not entirely fit into the bore on a larger-scale. To detect this a gross collision detection algorithm is executed which compares the examination volume and extent to the available bore diameter or, in general, the area not occupied or used by the system during acquisition. Here the 3D sensing capability is used to measure the setup in 3D. A critical capability of this algorithm may be to filter-out non-patient signals from patient signals (i.e., signals belonging to patient and setup on patient table) to avoid false positives. The following set of algorithms is proposed:
- Determine regions and contours in the image that do not fit the available space
- Determine the signal contour location and classify signal as being either related to patient exam setup or not.
- Determine signal motion correlation to e.g. relevant system actions such as table movements, use the correlation values to associate the signal with patient exam setup or not.
- Additionally used the body keypoint information: If the area does not fit the patient body, the signal is ignored or rated lower

The same overview network mentioned earlier also localizes relevant objects and accessories. Safety risks may not only be of mechanical but also of RF/thermal type. For reduction of RF/thermal safety incidents it is proposed to monitor closely the position and route of RF coil cables. Such cables must be positioned according to strict instructions by the manufacturer to prevent RF safety and thermal injury incidents. However, the correct cable routing may not be not a simple task and again cable deformation may happen spontaneously and out of sight of the operator. Also, remote scanner operation would require monitoring all cables before and during movement of the patient in the bore.

Another feature connected to RF safety is the constant monitoring of all RF coil positions with a camera. The comparison of the detected coils with the system reported coil connection status provides an immediate means to avoid unconnected coils in the examination setup.

Similar accessories such as the nurse call 600 (a device which the patient can use to trigger attention from the operator) are also monitored (Fig. 1 middle), as well as the proper location of the hearing protection and communication headset (504) in relation to the patient ears.

The above detected events may be potentially unsafe and this information (safety state) if detected with high confidence to:
- Provide information for resolving the safety events to the operator incl.
   - Spatial localization of the event
   - Type and context of the event
   - Estimated severity of the risk
   - Proposal for resolving
- Veto automated system behavior for a subset of events with high severity e.g.
   - Stop automatic table motion when fingers sticking outside of a safe area are detected, close to a pinching location
   - Slowing down the table when a collision/finger pinching event is likely to occur
   - Allow manual override only with controls close to the patient (e.g. via the thumb switch for table motion)
- Provide information for identifying resolving the safety events to the patient incl.
   - Automatic voice instruction/alert via speaker system
   - Showing the detected areas overlaid to the patient body to facilitate intuitive localization by the operator in real world.

If there are low confidence detections or undetectable body parts or objects, the camera may not be able to safely and adequately intervene with the workflow. For such situations a different system behavior can be used, e.g. if a hand is not detected by the camera (e.g. because the detection fails or because it is simply not visible at all), there could be a general recommendation to the operator to check the respective hand and make sure it is in safe position, protection devices have been applied etc.. Some of these situations can occur regularly due to the complexity of typical exam setups (e.g. hands covered by coils or blankets), blocking the system in such a situation would render the system unusable and must therefore be carefully avoided. Only high confidence detections can thus be used to reduce the safety incidence rate so that unjustified system blockage rate is reduced to low, user-acceptable levels.

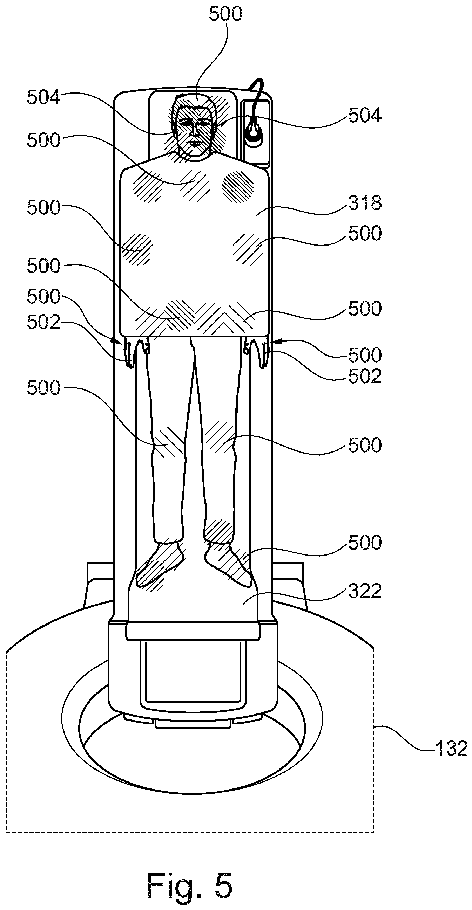

Fig. 5 illustrates an example of an object sub-image 132 that shows a subject 318 reposing on a subject support 320 for a magnetic resonance imaging system. A number of anatomical keypoint locators 500 are visible. The subject's hands 500 are in a safe position in terms of collision and finger pinching and a headset 504 is shown as being placed correctly. However, in this image, the nurse call is not seated in the patient's left hand correctly. Proper patient communication should be established prior to moving the subject support and the patient into the magnet.

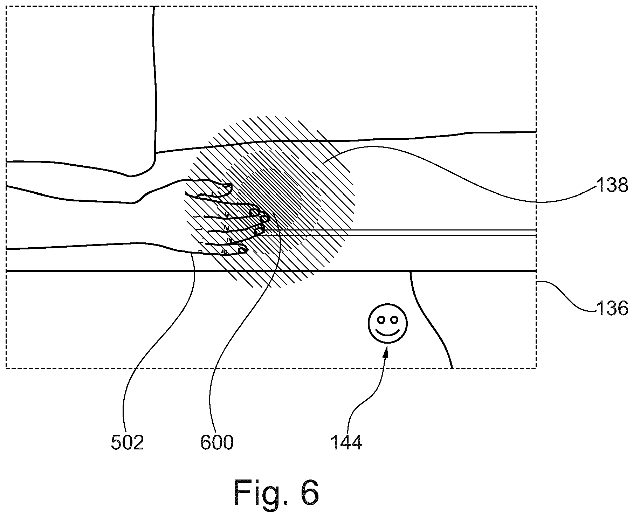

Fig. 6 shows an example of a search image 136. In this example a neural network or an item-specific neural network 126 that has been trained to detect and locate that the nurse call is within reach of the patient's hand. It can be seen that in the subject's hand 502 locator data 138 identifies the location of a nurse call 600.

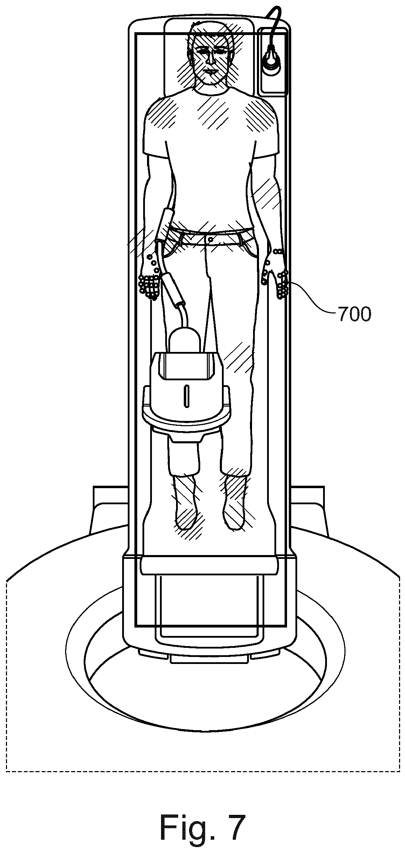

Fig. 7 depicts an image similar to that illustrated in Fig. 5. In this example there is a warning signal 700 that outlines the subject's hand as a warning that indicates a finger pinching risk the left hand. The table movement is unsafe when the hands get near the junction to the table motor stage, the bore and the trolley dock locations.

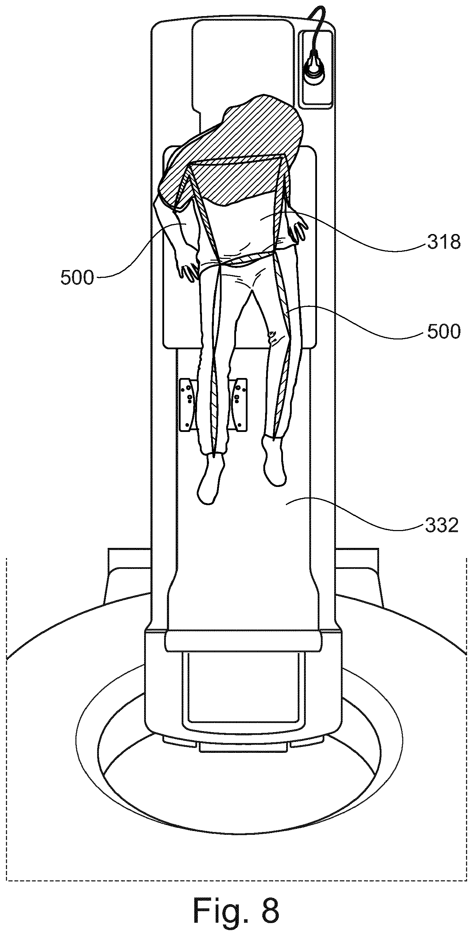

Fig. 8 illustrates the correct detection of patient pose not fitting the available examination space.

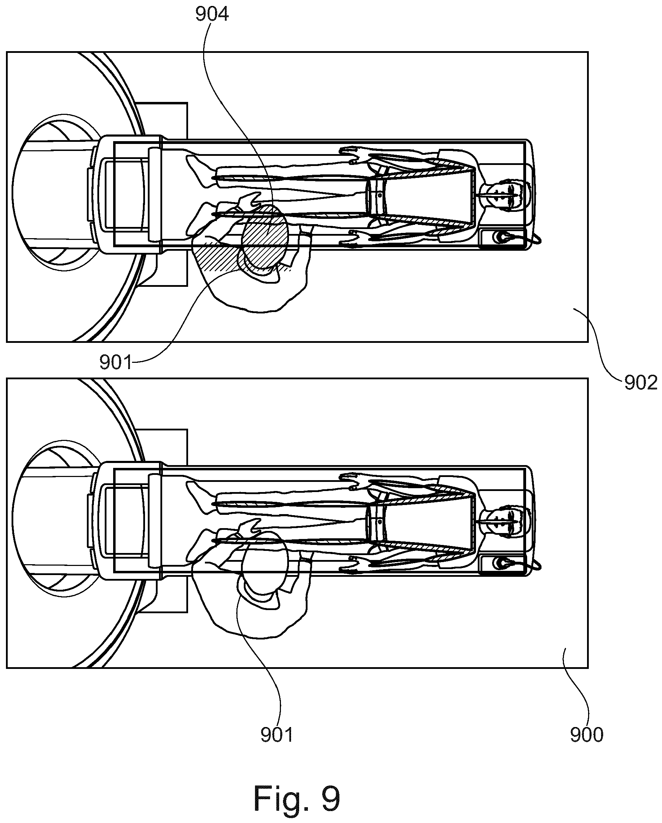

Fig. 9 illustrates the detection of spurious volumes that are not part of the patient examination setup. The image 900 has not had signals from the operator 901 removed. The image 902 illustrates an operator zone 904 where signals from the operator 901 have been identified and removed.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: computational system
- 106: hardware interface
- 108: user interface
- 110: memory
- 120: machine executable instructions
- 122: first neural network
- 124: object specific neural network
- 126: item specific neural network
- 128: overview image
- 130: object locators
- 132: object sub images
- 134: object positional data
- 136: multiple search images
- 138: locator data
- 140: safety state
- 142: predetermined criterion
- 144: warning signal
- 200: receive an overview image, wherein the overview image has an original resolution and is descriptive of a subject positioned on a subject support of a medical imaging system
- 202: receive object locators descriptive of a position and orientation of multiple predetermined objects in the overview image in response to inputting the overview image into a first neural network
- 204: construct an object sub image for each of the multiple predetermined objects using the object locator
- 206: receive object positional data descriptive of each of the multiple predetermined objects in response to inputting the object sub image for each of the multiple predetermined objects into an object specific neural network that is unique for each of the multiple predetermined objects
- 208: construct multiple search images using the object positional data for the multiple predetermined objects
- 210: receive locator data descriptive of a position of an item in response to inputting each of the multiple search images into an item specific neural network
- 212: detect a safety state by comparing the locator data and the object positional data of each of the predetermined objects with respect to a predetermined criterion
- 214: provide a warning signal if the safety state is detected
- 300: medical system
- 302: magnetic resonance imaging system
- 304: magnet
- 306: bore of magnet
- 308: imaging zone
- 310: magnetic field gradient coils
- 312: magnetic field gradient coil power supply
- 314: radio-frequency coil
- 316: transceiver
- 318: subject
- 320: subject support
- 322: table trolley
- 324: main motorized subject support
- 326: imaging device
- 330: pulse sequence commands
- 332: measured k-space data
- 334: magnetic resonance image
- 400: control the imaging device to acquire the overview image
- 500: anatomical keypoint locators
- 502: hands
- 504: headset
- 600: nurse call
- 700: warning signal
- 900: image
- 901: operator
- 902: image
- 904: operator zone

## Claims

1. A medical system (100, 300) comprising:
- a memory (110) storing machine executable instructions (120), a first neural network (122), an object specific neural network (124), and an item specific neural network (126);
- a computational system (104), wherein execution of the machine executable instructions causes the computational system to:
- receive (200) an overview image (128), wherein the overview image has an original resolution and is descriptive of a subject (318) positioned on a subject support (320) of a medical imaging system (302);
- receive (202) object locators (130) descriptive of a position and orientation of multiple predetermined objects in the overview image in response to inputting the overview image into the first neural network, wherein the first neural network is configured to process the overview image at a first resolution, wherein the first resolution is lower than the original resolution, wherein the multiple predetermined objects comprise the subject and the subject support;
- construct (204) an object sub image (132) for each of the multiple predetermined objects using the object locator, wherein each object sub image has a second resolution, wherein the second resolution is lower than the original resolution and higher than the first resolution;
- receive (206) object positional data (134) descriptive of each of the multiple predetermined objects in response to inputting the object sub image for each of the multiple predetermined objects into the object specific neural network that is unique for each of the multiple predetermined objects,
- construct (208) multiple search images (136) using the object positional data for the multiple predetermined objects, wherein the multiple search images have a third resolution that is higher than the second resolution, wherein the third resolution is less than or equal to the original resolution;
- receive (210) locator data (138) descriptive of a position of an item in response to inputting each of the multiple search images into the item specific neural network;
- detect (212) a safety state (140) by comparing the locator data and the object positional data of each of the predetermined objects with respect to a predetermined criterion; and
- provide (214) a warning signal (144, 700) if the safety state is detected.

2. The medical system of claim 1, wherein execution of the machine executable instructions causes the computational system to display a warning graphical user interface if the warning signal is provided, wherein the warning graphical user interface is configured to perform any one of the following:
- provide a spatial localization of a cause of the safety state,
- display a warning signal type and/or warning context using the predetermined criterion,
- display a severity ranking using the predetermined criterion,
- display operator instructions retrieved from an instruction database in response to a query using the predetermined criterion,
- provide a voice or audible alert,
- project a localized warning light onto the subject using a projector, wherein the localized warning light is configured to illuminate the item that triggered the safety state, and
- combinations thereof

3. The medical system of claim 1 or 2, wherein the object specific neural network for the subject is configured to output a set of anatomical keypoint locators (500), wherein at least a portion of the multiple search images are determined using a predetermined geometric relation to the set of anatomical keypoint locators.

4. The medical system of claim 3, wherein execution of the machine executable instructions further causes the computational system to:
- filter the locator data using a locator filter modified by the keypoint locators; and
- ignore locator data filtered by the locator filter.

5. The medical system of claim 3, 4, or 5, wherein any one of the following:
- the item is a nurse call (600) squeeze bulb;
- the item is a headset (504) and/or ear plugs, wherein the multiple search images comprise a head region of the subject, and wherein the locator data is descriptive of a position of the item relative to a center locator of ears of the subject.
- the item is a conductive cable, wherein the multiple predetermined objects comprises a magnetic resonance imaging coil and/or a wired apparatus, wherein the multiple predetermined objects further comprises at least one magnetic resonance transmit body coil element, wherein the multiple search images comprise regions adjacent to the magnetic resonance imaging coil and/or the wired apparatus, wherein the multiple search images further comprise regions adjacent to the at least one transmit body coil element, wherein the multiple search regions further comprise additional regions with a predefined location relative to the set of anatomical keypoint locators; and
- the item is a hand, wherein the warning signal is configured to provide a dangerous hand position warning signal if the hand is located at a pinch location or a current loop causing position.

6. The medical system of any one of the preceding claims, wherein the multiple predetermined objects comprise a main motorized patient support (324), wherein the item is an MR table trolley (322), wherein the multiple search images are positioned to cover a rim of the main motorized patient support of the MR to check for potential collisions with the MR table trolley when automatically moving the main motorized patient support.

7. The medical system of any one the preceding claims, wherein the item is any one of the following:
- a cushion;
- a cable;
- a breathing tube;
- an anatomical structure;
- an intravenous supply;
- the item is a MR conditional device, wherein the location of the multiple search images are derived from a chosen magnetic resonance imaging protocol,
- a magnetic field sensitive device designed to be kept within a maximum B0 field strength, wherein the search images are located at regions where the B0 field strength is above a predetermined threshold or up to the magnetic isoline;
- a proximity sensitive device designed to be kept a minimum distance from a second device, wherein the second device is one of the multiple predetermined objects, wherein the multiple search images are located at predefined locations relative to the object positional data of the second device; and
- combinations thereof.

8. The medical system of any one of the preceding claims, wherein the multiple predetermined objects comprise one or more magnetic resonance imaging coils, wherein the locator data comprises radio-frequency cable location data,
wherein execution of the machine executable instructions further causes the computational system to determine the safety state by performing any one of the following:
- comparing the object positional data of the one or more magnetic resonance imaging coils and the radio-frequency location data to a set of allowable radio-frequency routing paths,
- comparing the object positional data of the one or more magnetic resonance imaging coils with a set of reported magnetic resonance imaging coil connections; and
- combinations thereof.

9. The medical system of any one of the preceding claims: wherein the overview image is any one of the following: an optical image, a color optical image, and a three-dimensional image.

10. The medical system of any one of the preceding claims, wherein the medical system further comprises a medical imaging system (302) with the subject support, wherein the medical system further comprises an imaging device (326) configured to acquire the overview image by imaging at least the subject support, wherein execution of the machine executable instructions further causes the computational system to control (400) the imaging device to acquire the overview image.

11. The medical system of claim 10, wherein the imaging device is a three-dimensional camera, wherein execution of the machine executable instructions further causes the computational system to perform any one of the following:
- compare spatial contours of the item to a predetermined volume to detect a collision condition,
- use contour data from the three-dimensional camera to filter out and ignore locator data as being non-examination setup related,
- determine signal motion correlation with subject support motion to filter out and ignore locator data as being non-examination setup related, and
- combinations thereof.

12. The medical system of any one of the preceding claims, wherein the multiple predetermined objects comprises an operator (901), wherein execution of the machine executable instructions further causes the computational system to:
- identify an operator zone (904) within the overview image using an object locator of the operator; and
- remove and/or ignore locator data and/or object positional data within the operator zone.

13. The medical system of any one of the preceding claims, wherein the overview image is received as an image stream with a frame rate, wherein the first neural network is configured to process the overview image equal to or at higher rate than the frame rate, wherein execution of the machine executable instruction causes the computational system to repeatedly input the overview image to obtain the object locators at the frame rate.

14. A computer program comprising machine executable instruction (120), a first neural network (122), an object specific neural network (124), and an item specific neural network (126), wherein execution of the machine executable instructions causes the computational system to:
- receive (200) an overview image (128), wherein the overview image has an original resolution and is descriptive of a subject positioned on a subject support of a medical imaging system;
- receive (202) object locators (130) descriptive of a position and orientation of multiple predetermined objects in the overview image in response to inputting the overview image into the first neural network, wherein the first neural network is configured to process the overview image at a first resolution, wherein the first resolution is lower than the original resolution, wherein the multiple predetermined objects comprise the subject and the subject support;
- construct (204) an object sub image (132) for each of the multiple predetermined objects using the object locator, wherein each object sub image has a second resolution, wherein the second resolution is lower than the original resolution and higher than the first resolution;
- receive (206) object positional data (134) descriptive of each of the multiple predetermined objects in response to inputting the object sub image for each of the multiple predetermined objects into an object specific neural network that is unique for each of the multiple predetermined objects,
- construct (208) multiple search images (136) using the object positional data for the multiple predetermined objects, wherein the multiple search images have a third resolution that is higher than the second resolution, wherein the third resolution is less than or equal to the original resolution;
- receive (210) locator data (138) descriptive of a position of an item in response to inputting each of the multiple search images into an item specific neural network;
- detect (212) a safety state (140) by comparing the locator data and the object positional data of each of the predetermined objects with respect to a predetermined criterion; and
- provide (214) a warning signal (144, 700) if the safety state is detected.

15. A computer implemented method, wherein the method comprises:
- receiving (200) an overview image (128), wherein the overview image has an original resolution and is descriptive of a subject positioned on a subject support of a medical imaging system;
- receiving (202) object locators (130) descriptive of a position and orientation of multiple predetermined objects in the overview image in response to inputting the overview image into a first neural network (122), wherein the first neural network is configured to process the overview image at a first resolution, wherein the first resolution is lower than the original resolution, wherein the multiple predetermined objects comprise the subject and the subject support;
- constructing (204) an object sub image (132) for each of the multiple predetermined objects using the object locator, wherein each object sub image has a second resolution, wherein the second resolution is lower than the original resolution and higher than the first resolution;
- receiving (206) object positional data (134) descriptive of each of the multiple predetermined objects in response to inputting the object sub image for each of the multiple predetermined objects into an object specific neural network (124) that is unique for each of the multiple predetermined objects,
- constructing (208) multiple search images (136) using the object positional data for the multiple predetermined objects, wherein the multiple search images have a third resolution that is higher than the second resolution, wherein the third resolution is less than or equal to the original resolution;
- receiving (210) locator data (138) descriptive of a position of an item in response to inputting each of the multiple search images into an item specific neural network (126);
- detecting (212) a safety state (140) by comparing the locator data and the object positional data of each of the predetermined objects with respect to a predetermined criterion; and
- providing (214) a warning signal (144, 700) if the safety state is detected.
